# EUROPÄISCHE PATENTSCHRIFT

(11) **EP 0 412 968 B1**
(45) Veröffentlichungstag und Bekanntmachung des Hinweises auf die Patenterteilung: **03.11.1993**
(21) Anmeldenummer: 89901714.9
(22) Anmeldetag: 30.01.1989
(51) Int. Cl.: A61M 5/315

(54) **SPRITZE, INSBESONDERE FÜR DEN MEDIZINISCHEN GEBRAUCH**
SYRINGE, IN PARTICULAR FOR MEDICAL USE
SERINGUE, NOTAMMENT A USAGE MEDICAL

(30) Priorität: 08.04.1988 DE 8804656 U; 18.08.1988 DE 3828127
(43) Veröffentlichungstag der Anmeldung: 20.02.1991
(73) Patentinhaber: BADER, Mohandes, D-24536 Neumünster (DE)
(72) Erfinder: BADER, Mohandes, D-24536 Neumünster (DE)
(74) Vertreter: MEISSNER, BOLTE & PARTNER
(86) Internationale Anmeldenummer: EP8900076
(87) Internationale Veröffentlichungsnummer: WO8909630

(56) Entgegenhaltungen:
- AU-B-16 859
- DE-C- 331 326
- GB-A-20 158 83
- US-A-37 994 11
- US-A-40 304 98

## Beschreibung

Die Erfindung betrifft eine Spritze, insbesondere für den medizinischen Gebrauch, gemäß dem Oberbegriff des Anspruches ].

Derartige Spritzen sind in der Medizintechnik allgemein bekannt, z.B. aus der GB-A-20 15 883 oder AU-A-16 859. Die Spritze gemäß der GB-A-20 15 883 entspricht im wesentlichen der Konstruktion gemäß der DE-B-29 09 002. Diese bekannten Spritzen sind zwar nur für einen einmaligen Gebrauch bestimmt, wobei zu diesem Zweck die Innenwand des Zylinders mindestens eine ringförmige senkrecht zur Zylinderachse angeordnete Nut oder Hinterschneidung aufweist, in die nach vollständig erfolgter Injektion wenigstens ein radial nach außen drängendes Kolbenteil in der Ausstoß-Endstellung des Kolbens im Zylinder nicht mehr zurückziehbar einrastet. Um bei dieser Injektionsspritze eine Mehrfachverwendung zu verhindern, ist es also unbedingt erforderlich, daß der Kolben bis in seine Ausstoß-Endstellung geschoben und eingerastet ist. Bei geschickter Handhabung der Kolbenstange ist eine Mehrfachverwendung der bekannten Spritze ohne weiteres möglich. Es muß lediglich darauf geachtet werden, daß der Kolben nicht bis in seine vorderste Stellung geschoben wird. Zu diesem Zweck braucht man außerhalb des Zylinders zwischen diesem und dem am freien Ende der Kolbenstange angeordneten Betätigungsteil um die Kolbenstange herum lediglich einen Distanzring anzubringen. Demnach bietet diese bekannte Injektionsspritze keine Gewähr dafür, daß sie nicht mehrfach verwendet wird. Im Hinblick auf die zunehmende Gefahr einer Aids-Ansteckung durch Mehrfachverwendung von Injektionsspritzen, insbesondere in unterentwickelten Ländern, sind die vorgenannten Spritzen-Konstruktionen nicht brauchbar.

Bei der Konstruktion nach der AU-A-16 859 ist nur eine portionsweise Verabreichung des Spritzeninhalts möglich. Des weiteren ist nicht erkennbar, daß bei dieser bekannten Konstruktion ein erster Saughub möglich ist. Die Befüllung des Spritzenzylinders erscheint wenig praktikabel und darüberhinaus höchst kontaminationsgefährdet. Auch ist die Dichtigkeit zwischen Kolben und Kolbenstange bei der bekannten Konstruktion höchst zweifelhaft, so daß auch darüber Kontaminationsgefahr besteht. Die Konstruktion nach der AU-A-16 859 ist daher nicht sehr praktikabel. Sie hat auch keinen Eingang in die Praxis gefunden, obwohl diese Konstruktion bereits seit 1934 bekannt ist.

Der vorliegenden Erfindung liegt die Aufgabe zugrunde, eine Spritze der eingangs genannten Art derart weiterzubilden, daß nach Einmalgebrauch eine Weiterverwendung mit Sicherheit ausgeschlossen ist, wobei insbesondere auch Manipulationen für eine Mehrfachverwendung nicht möglich sein sollen.

Diese Aufgabe wird durch die kennzeichnenden Merkmale des Patentanspruches 1 gelöst. Bevorzugte konstruktive Details der erfindungsgemäßen Konstruktion sind in den Unteransprüchen beschrieben.

Der Kern der vorliegenden Erfindung liegt also darin, daß ein gesondertes Sperrmittel in Form einer von der Kolbenstange durchsetzten Sperrhülse vorgesehen ist, wobei die Zusammenwirkung zwischen Sperrhülse und Kolbenstange derart ist, daß das Spritzenvolumen nur ein einziges Mal verabreichbar ist, und zwar auch dann, wenn die Kolbenstange in mehreren Teilhüben bewegt wird. Aufgrund der Trennung von Kolben, Kolbenstange einerseits und Sperrmittel andererseits treten auch die oben im Zusammenhang mit der Konstruktion nach der AU-A-16 859 dargelegten Dichtigkeits- und Kontaminationsprobleme nicht auf.

Eine zusätzliche Sicherheit gegen eine Mehrfachverwendung stellen die Merkmale der Ansprüche 12 ff dar. Diese Merkmale führen zu einer Funktionszerstörung der Spritze, so daß auch unter Ausübung von Gewalt die Mehrfachverwendung der Spritze ausgeschlossen ist.

Vorzugsweise ist die Injektionsnadel fest mit dem Spritzenzylinder verbunden, so daß auch eine Mehrfachverwendung der Injektionsnadeln unmöglich wird.

Weitere Merkmale der Erfindung werden nachstehend am Beispiel der in den Zeichnungen dargestellten Ausführungsbeispiele näher erläutert. Es zeigen:
- Fig. 1: eine erste Ausführungsform einer Spritze in einer Explosionsdarstellung, teilweise im Schnitt;
- Fig. 2: die Spritze nach Fig. 1 in zusammengebauter Stellung vor Ingebrauchnahme mit eingeschobenem Kolben im Längsschnitt;
- Fig. 3: die Spritze nach Fig. 2 nach einem Saughub mit ausgezogenem Kolben im Längsschnitt;
- Fig. 4: die Spritze nach Fig. 3 nach einem Leerhub mit gegen erneutes Herausziehen gesperrtem Kolben im Längsschnitt;
- Fig. 5a bis 5l: mögliche Querschnittsformen der Kolbenstange für eine Spritze nach den Fig. 1 bis 4;
- Fig. 6: eine als Sperrmittel dienende Sperrhülse in Zusammenwirkung mit der Kolbenstange als vergrößerte Einzelheit im Längsschnitt;
- Fig. 7: eine weitere Ausführungsform eines Sperrglieds in schematischer Seitenansicht, teilweise im Schnitt;
- Fig. 8: eine weitere Ausführungsform einer Spritze gemäß Erfindung im schematischen Längsschnitt vor Ingebrauchnahme mit eingeschobenem Kolben;
- Fig. 9: die der Spritze gemäß Fig. 8 zugeordnete Sperrhülse im Schnitt und vergrößertem Maßstab;
- Fig. 10: die Sperrhülse nach Fig. 9 in Draufsicht;
- Fig. 11: die bei der Spritze gemäß Fig. 8 verwendete Kolbenstange in Seitenansicht und vergrößertem Maßstab;
- Fig. 12: einen Teil der Kolbenstange gemäß Fig. 11 im Schnitt längs Linie XVI-XVI in Fig. 11;
- Fig. 13: Draufsicht auf die Kolbenstange gemäß Fig. 11 bzw. deren Betätigungsscheibe;
- Fig. 14: eine fünfte Ausführungsform einer Spritze gemiß Erfindung im schematischen Längsschnitt vor Ingebrauchnahme mit eingeschobenem Kolben;
- Fig. 15a: das Sperrglied für die Ausführungsform gemäß Fig. 14 im Längsschnitt und vergrößertem Maßstab;
- Fig. 15b: das Sperrglied gemäß Fig. 15a in Draufsicht;
- Fig. 16a: eine alternative Ausführung des Sperrglieds in einer Darstellung entsprechend Fig. 15a;
- Fig. 16b: das Sperrglied gemäß Fig. 16a in Ansicht von unten;
- Fig. 17a: eine weitere Abwandlung des Sperrglieds in einer Darstellung entsprechend Fig. 15a oder 16a; und
- Fig. 17b: das Sperrglied gemäß Fig. 17a in Ansicht von unten.

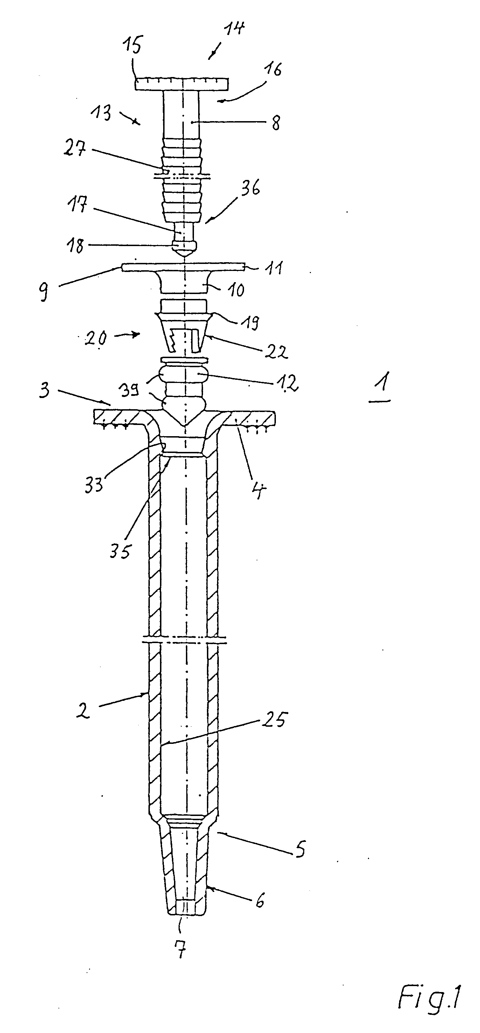

Die Spritze nach den Fig. 1 bis 4 besteht aus einem Zylinder 2, einer Kolbenstange 13, einer Sperrhülse 19, einem Kolben 12 mit zwei im axialen Abstand voneinander angeordneten Ringlippendichtungen 39 und einem Kolbenstangen-Führungsteil 9. Der Zylinder weist an seinem rückwärtigen Öffnungsabschnitt 3 einen radial vorstehenden Flansch 4 auf. Am vorderen Endabschnitt 5 des Zylinders 2 ist ein Stutzen 6 mit einer axialen Öffnung 7 ausgebildet. In dieser ist eine nicht naher dargestellte Injektionsnadel oder Kanüle oder dgl. Saug- und/oder Verabreichungsteil unlösbar befestigt, insbesondere eingeklebt.

Das Führungsteil 9 besteht aus einer Führungsbuchse 10, an die ein Flansch 11 angeformt ist. In der Führungsbuchse 10 ist die Kolbenstange 13 geführt. Der Flansch 11 liegt im eingebauten Zustand des Führungsteils 9 auf dem Flansch 4 des Zylinders 2 auf. Die Verbindung des Führungsteils 9 mit dem Zylinder 2 kann durch Klebung, Schweißen oder Einpressen der Führungsbuchse 10 in den Zylinder 2 erfolgen.

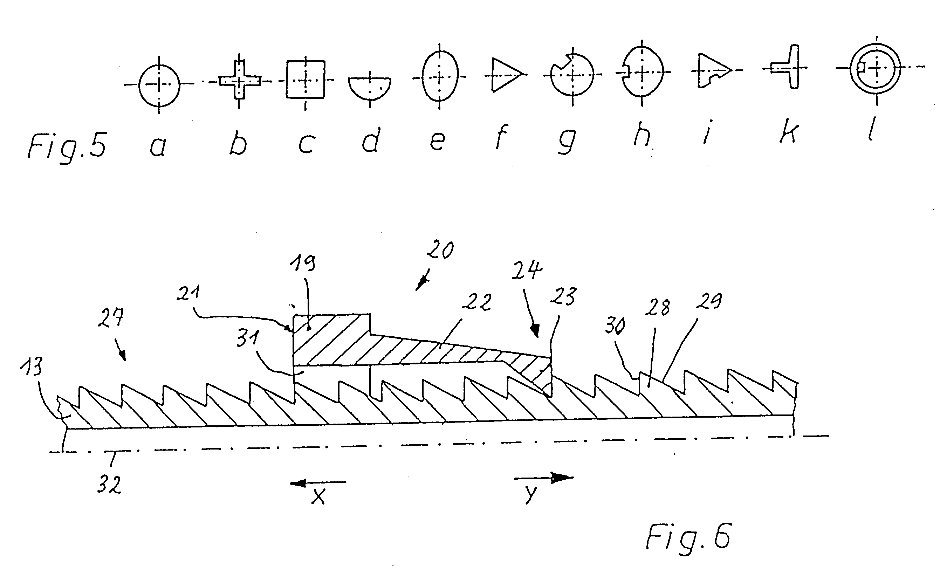

Die Kolbenstange 13 besteht aus einem länglichen Mittelstück 8, an dessen einem Ende eine als Betätigungsscheibe 15 ausgebildete Handhabe 14 und an dessen anderem Ende 36 eine Einschnürung 17 angeformt bzw. ausgebildet ist. Die Einschnürung 17 wird demnach zum einen durch das durchmessergrößere Mittelstück und zum anderen durch eine endseitige Verdickung 18 begrenzt. Die Einschnürung 17 dient zur Halterung des aus einem Elastomer bestehenden Kolbens 12, der beim Zusammenbau der Spritze über die Verdickung 18 auf die Einschnürung 17 geschoben wird. Auf dem Umfang des Mittelstücks 8 der Kolbenstange 13 ist eine Zahnung 27 ausgebildet. Wie in Fig. 6 näher dargestellt, sind die Zähne 28 sägezahnartig ausgebildet und angeordnet, wobei die der Verdickung 18 bzw. dem Kolben 12 zugewandten Zahnflanken 29 schiefwinklig zur Kolbenstangenlängsachse 32 und die der Handhabe 14 zugewandten Zahnflanken 30 rechtwinklig zur Kolbenstangenlängsachse 32 ausgebildet sind. Mittels der Zahnung 27 kann die Kolbenstange 13 mit dem als Sperrhülse 19 ausgebildeten Sperrglied 20 in mechanischen Wirkeingriff gebracht werden.

An der Sperrhülse 19 sind elastische Arretierungszungen 22 ausgebildet. Deren freie Endabschnitte 24 sind als nach innen gerichtete Sperrklinken 23 ausgeformt. Diese Sperrklinken 23 sind bei zusammengebauter Spritze mit der Zahnung 27 der Kolbenstange 13 in Wirkeingriff. Wesentlich für die Funktion der Sperrhülse ist, daß deren Reibungswiderstand an der Innenwand 25 des Zylinders 2 größer ist als die auf die schiefwinksligen Zahnflanken 29 einwirkenden Klemmkräfte der Sperrklinke 23 der Arretierungszungen 22.

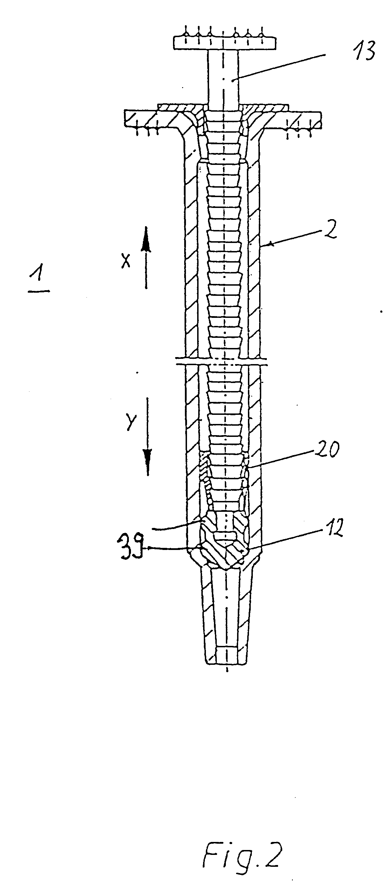

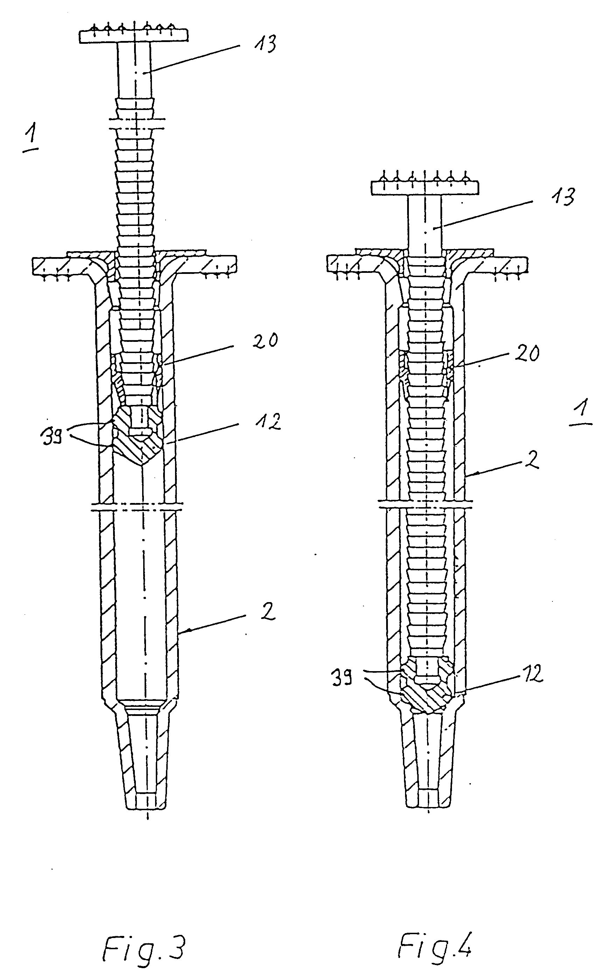

Der Zusammenbau der Spritze 1 geschieht wie folgt:
Zunächst wird der Kolben 12 in den Zylinder 2 bis zum Bereich des Stutzens 6 geschoben. Dann wird die Sperrhülse 19 in den Zylinder 2 eingeschoben bis sie am Kolben 12 anliegt. Danach wird das Führungsteil 9 mit dem Zylinder 2 verbunden. Dann wird die Kolbenstange 13 in den Zylinder 2 eingeschoben und gegen den Kolben 12 gedrückt bis dieser über die Verdickung 18 rutscht und an der Einschnürung 17 befestigt ist. In dieser Stellung, die in Fig. 2 dargestellt ist, kann die Spritze 1 in Gebrauch genommen werden.

Wenn die Kolbenstange 13 beispielsweise zur Blutentnahme oder zur Aufnahme einer medizinischen Lösung aus dem Zylinder 2 gezogen wird, rasten die Sperrklingen 23 in die Zahnung 27 ein und liegen an einer der Zahnflanken 30 an. Hierdurch wird die Sperrhülse 19 mit der Kolbenstange 13 in Richtung des Öffnungsabschnitts 3 gezogen. Bei einer Umkehr der Kolbenverschiebung in Ausstoßrichtung y, also in den Zylinder 2 hinein, werden die Sperrklinken 22 entriegelt, und die Sperrhülse 19 verbleibt in der Stellung im Zylinder 2 lagefixiert, die sie bei der vorhergehenden Hubbewegung des Kolbens 12 bzw. der Kolbenstange 13 in Hubrichtung x erreicht hatte. Je nach Größe dieser Hubbewegung kann die Stellung, in der die Sperrhülse 19 durch Reibwirkung an der Innenwand 25 des Zylinders 2 festklemmt, unterschiedlich sein. In der äußersten Endstellung liegt die Sperrhülse an einer am Öffnungsabschnitt 3 des Zylinders 2 ausgebildeten Einziehung 33 an, die als Auszugssperre 35 dient und bewirkt, daß die Spritze 1 nicht zerstörungsfrei demontiert werden kann (Fig. 3). Bei der genannten Umkehr der Kolbenverschiebung in Ausstoßrichtung y gleiten die Sperrklinken 23 über die schiefwinklig angeordneten Zahnflanken 29 der Zähne 28 der Zahnung 27, ohne daß die Sperrhülse 19 verschoben wird. Bei einer erneuten Betätigung des Kolbens 12 bzw. der Kolbenstange 13 in Saughubrichtung x kann die Sperrhülse 19 durch die Klemmkraft der aufliegenden Sperrklingen 23 nur dann weiter in Richtung zur Einziehung 33 verschoben werden, wenn sie sich vorher im Abstand von dieser befand. Der Kolben 12 bzw. die Kolbenstange 13 kann somit nur einmal aus der in Fig. 2 dargestellten Ingebrauchnahmestellung über den vollen Kolbenhub in Hubrichtung x in die in Fig. 3 desgestellte Endstellung Gezogen werden. In dieser ist der Zylinder 2 mit dem maximal möglichen Volumen an gewünschter flüssiger Substanz, z. B. Blut oder medizinischer Lösung, gefüllt. Die Wirkung der Sperrhülse 19 hat zur Folge, daß der Kolben 12 bzw. die Kolbenstange 13 zwar aus der in Fig. 3 dargestellten Stellung wieder in den Zylinder 2 hineingeschoben werden kann, ein erneutes Herausziehen in Hubrichtung x aber nicht mehr möglich ist, daß dieses durch die an einer Zahnflanke 30 anliegenden Sperrklinken 23 verhindert wird. Es ist somit zwar noch ein Leerhub in eine Kolbenstellung entsprechend Fig. 4 möglich, aber kein erneuter Saughub. Somit ist sichergestellt, daß die Spritze nur für eine bestimmungsgemäße einmalige Verwendung nutzbar ist. Das Zylindervolumen ist nur ein einziges Mal verabreichbar. Auch durch Manipulationen an der Spritze ist es nicht möglich, mehr als das maximale Zylindervolumen zu verabreichen.

Ein als Sperrhülse 19 ausgebildetes Sperrglied 20 ist bei den verschiedensten Querschnittsformen der Kolbenstange 13 einsetzoar. In den Fig. 5a bis 5l sind ohne Anspruch auf Vollständigkeit mögliche Querschnittsformen der Kolbenstange 13 dargestellt. Es ist bei diesen Querschnitten lediglich erforderlich, die Anordnung der Arretierungszungen 22 mit den Sperrklinken 23 an der Sperrhülse 19 entsprechend an die Querschnittsform der Kolbenstange 13 anzupassen.

Die beschriebene Spritze 1 kann in verschiedenen Größen und in allen bekannten Bauarten ausgebildet werden.

Es ist auch möglich, das Sperrglied 20 so auszubilden, daß auf eine Zahnung 27 an der Kolbenstange 13 verzichtet werden kann. So kann z. B. an der Sperrhülse 19 eine elastische Klemmzunge 41 aus Metall oder dgl. angeformt sein, deren freier Endabschnitt in der Einbaustellung bei der Spritze 1 zum Stutzen 6 gerichtet ist und die unter Federdruck auf der Kolbenstange 13 aufliegt. Die Federkraft der Klemmzunge 21 wird dabei so dimensioniert, daß sie kleiner ist als der Reibungswiderstand der Sperrhülse 19 an der Innenwand 25 des Zylinders 2. Beim Einschieben der Kolbenstange 13 in den Zylinder wird durch die Anpreßkraft die Klemmzunge nach außen gedrückt und die Sperrhülse 19 verbleibt in ihrer Stellung. Beim Zurückziehen des Kolbens bzw. der Kolbenstange 13 wird dann die Sperrhülse 19, wie oben beschrieben, in Richtung zum Öffnungsabschnitt 3 des Zylinders 2 bis maximal zur Einziehung 33 geschoben. Hiernach kann der Kolben 12 bzw. die Kolbenstange 13 zwar wieder in den Zylinder 2 hineingeschoben, nicht aber mehr herausgezogen werden.

Es ist auch möglich, zur Vereinfachung des konstruktiven Aufbaus der Spritze 1 auf das Führungsteil 9 zu verzichten, was insbesondere bei kleineren Spritzen zweckmäßig ist.

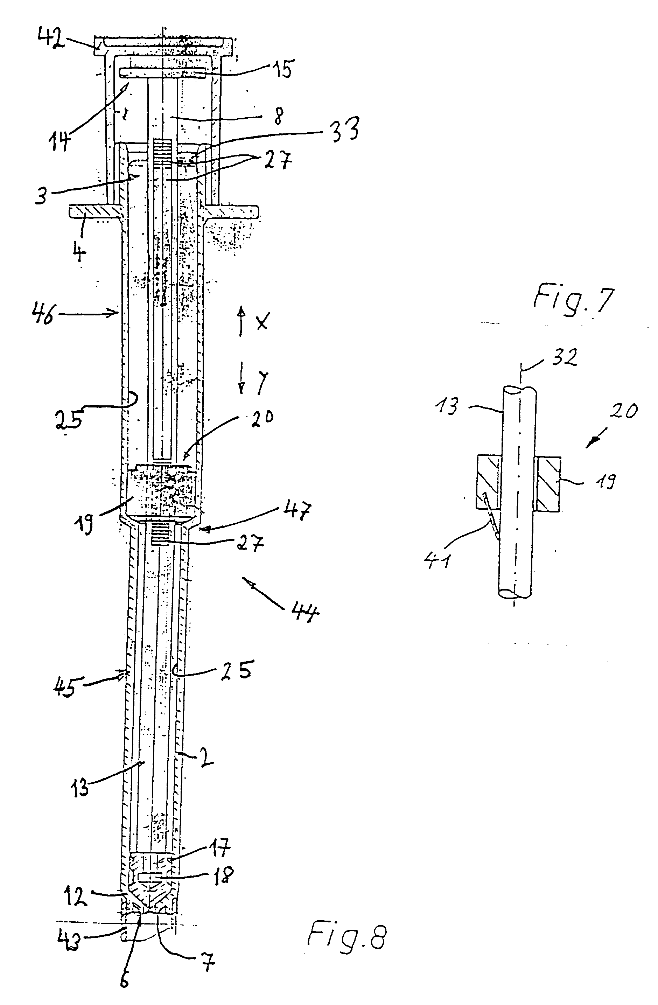

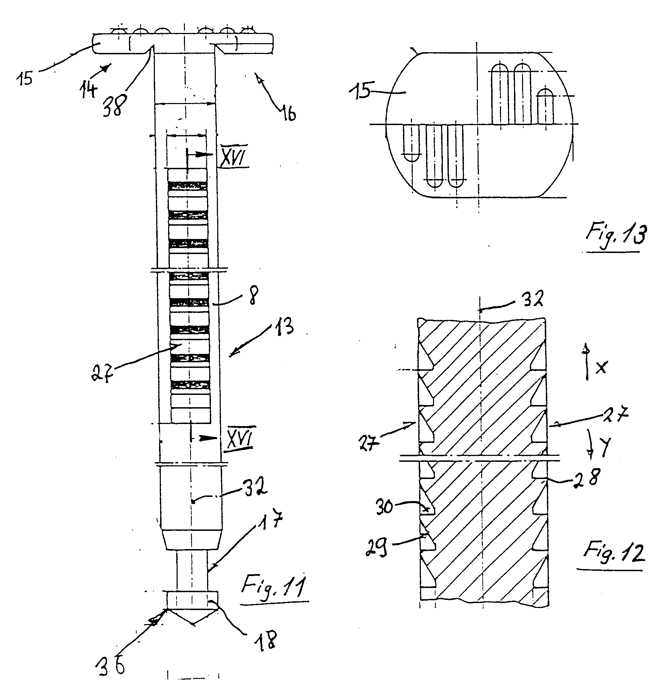

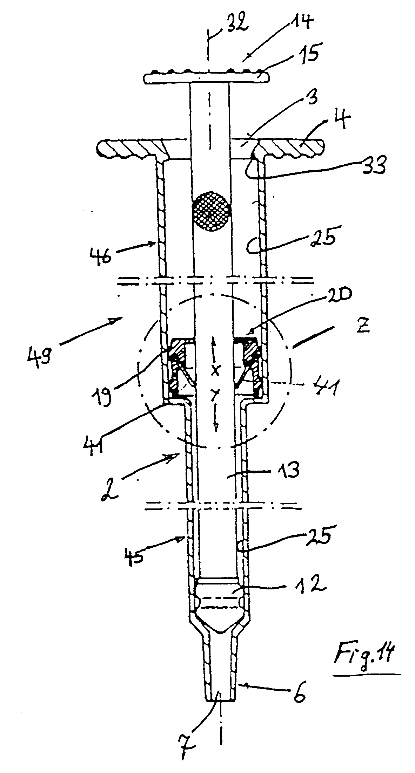

Anhand der Figuren 8 bis 13 soll nun eine weitere bevorzugte Ausführungsform einer erfindungsgemäß ausgebildeten Spritze beschrieben werden, wobei Bauelemente, die bereits anhand der Spritze 1 beschrieben sind, mit jeweils denselben Bezugsziffern gekennzeichnet sind. Dementsprechend besteht die in Fig. 8 im schematischen Längsschnitt dargestellte Spritze 44 aus einem Zylinder 2 mit einem vorderen bzw. in Fig. 8 unteren Zylinderabschnitt 45 und einem dem rückwärtigen Ende der Spritze zugewandten bzw. in Fig. 8 oberen Abschnitt 46, welcher einen etwas größeren Durchmesser aufweist als der Zylinderabschnitt 45. Die Ringstufe zwischen den beiden Zylinderabschnitten 45, 46 ist in Fig. 8 mit der Bezugsziffer 47 gekennzeichnet. Im in Fig. 8 unteren Zylinderabschnitt ist dar Kolben 12 gegenüber der Innenwand 25 fluiddicht verschieblich gelagert, während im oberen Zylinderabschnitt 46 die Axialbewegung des als Sperrhülse 19 ausgebildeten Sperrglieds 20 erfolgt. Beide Zylinderabschnitte weisen dieselbe wirksame Länge auf. Ein oberes Führungsteil 9 ist weggelassen. Entsprechend den Fig. 11 bis 13 besteht die Kolbenstange 13 ähnlich wie bei der Ausführungsform nach den Fig. 1 ff aus einem länglichen Mittelstück 8, an dessen einem Ende 16 eine als Betätigungsscheibe 15 ausgebildete Handhabe 14 und an dessen anderem Ende 36 die Einschnürung 17 sowie Verdickung 18 ausgebildet sind. Der Kolben 12 ist ebenfalls wie bei den voranbeschriebenen Ausführungsbeispielen ausgebildet und am vorderen Ende der Kolbenstange 13 fixiert. Die Ausführungsform mit einem gesonderten Führungszylinder 46 für die Sperrhülse 19, welcher nicht unbedingt mit einem größeren Durchmesser als der dem Kolben 12 zugeordnete Führungszylinder 45 ausgebildet sein muß, hat den Vorteil gegenüber den vorbeschriebenen Ausführungsformen, daß die dem Kolben 12 zugeordnete Zylinderlaufbahn 25 durch mechanische Einwirkung der Sperrhülse 19 bzw. deren Sperrelemente nicht beschädigt wird, wodurch Undichtigkeiten auftreten können. Dies gilt vor allem bei Verwendung von Sperrhülsen 19 mit metallischen Klemmzungen, die auf die Zylinderwand 25 einwirken.

Die Ausführungsform mit vergrößertem Führungszylinder 46 wird bevorzugt verwendet für die Aufnahme von Sperrgliedern 20 mit Metallklemmzungen, die sperrend auf die Zylinderwand einwirken.

In jedem Fall schaben die metallischen Klemmzungen an der Zylinderwand bei Verwendung der Spritze mit der Folge, daß Kunststoffteilchen von der Zylinderwand 25 abgeschabt werden. Diese könnten bei zum Teil gemeinsamer Zylinderlaufbahn 25 für Sperrglied 20 und Kolben 12 zwischen diesen und die Zylinderwand hindurch in das zu verabreichende medizinische Medium und damit in die Blutbahn eines Patienten gelangen. Die nachteiligen Folgen sind augenscheinlich.

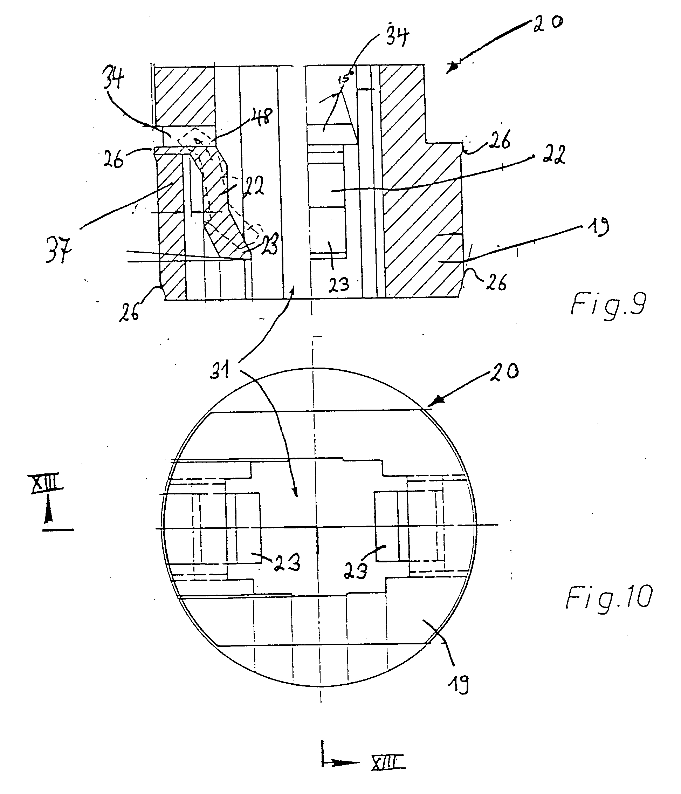

Wie in den Fig. 11 und 12 näher dargestellt, ist über eine Teillänge der Kolbenstange 13 eine diametrale Zahnung 27 ausgebildet, wobei die Zähne 28 sägezahnartig angeordnet sind derart, daß die der Einschnürung 17 zugewandten Zahnflanken 29 schiefwinklig zur Kolbenstangenlängsachse 32 und die der handhabe 14 zugewandten Zahnflanken 30 rechtwinklig zur Kolbenlängsachse 32 ausgebildet sind. Mittels der Zahnung 27 kann die Kolbenstange 13 mit dem als Sperrhülse 19 gemäß den Fig. 9 und 10 ausgebildeten Sperrglied 20 in mechanischen Wirkeingriff gebracht werden.

An der im oberen Zylinderabschnitt 46 verschieblich gelagerten Sperrhülse 19 sind entsprechend den Fig. 9 und 10 diametral zueinander angeordnete elastische Arretierungszungen 22 ausgebildet. Deren freie Endabschnitte sind ähnlich wie bei der Ausführungsform nach den Fig. 1 bis 6 als nach innen gerichtete Sperrklinken 23 ausgeformt. Diese Sperrklinken 23 sind bei zusammengebauter Spritze mit der diametral angeordneten Zahnung 27 der Kolbenstange 13 in Wirkeingriff. Wesentlich für die Funktion der Sperrhülse 19 ist, daß deren Reibungswiderstand an der Innenwand 25 des in Fig. 8 oberen Zylinderabschnitts 46 größer ist als die auf die schiefwinkligen Zahnflanken 29 einwirkenden Klemmkräfte der Sperrklinken 23 der Arretierungszungen 22. Zur Erhöhung des notwendigen Reibungswiderstandes zwischen Sperrhülse 19 und Innenwand bzw. Zylinderlaufbahn 25 können an den Sperrhülsen 19 gesonderte Stützringvorsprünge 26 angeformt sein (siehe Fig. 9). Der Zusammenbau der Spritze 44 erfolgt ähnich wie der der Spritze 1, und zwar wie folgt:
Zunächst wird der Kolben 12 in den Zylinder 2, und zwar in den in Fig. 8 unteren Zylinderabschnitt 45 bis zum Bereich der Bodenöffnung 7 geschoben. Dann wird die Sperrhülse 19 in den in Fig. 8 oberen Zylinderabschnitt 46 eingeschoben, bis sie an der Ringstufe 47 zwischen oberem und unterem Zylinderabschnitt anliegt. Danach wird die Kolbenstange 27 in den Zylinder 2 eingeschoben, und zwar durch das Sperrglied 19 hindurch, und gegen die Elastomer-Kolben 12 gedrückt bis dieser über die Verdickung 18 rutscht und an der Einschnürung 17 somit befestigt ist. In dieser Stellung, die in Fig. 8 dargestellt ist, kann die Spritze 44 in Gebrauch genommen werden. Wenn der Kolben 12 bzw. die Kolbenstange 13 beispielsweise zum Ansaugen einer medizinischen Lösung, insbesondere eines Impfstoffes, aus dem Zylinder 2 gezogen wird, rasten die Sperrklinken 23 in die Zahnung 27 ein und liegen an einer der Zahnflanken 30 an. Hierdurch wird die Sperrhülse 19 mit der Kolbenstange 13 in Richtung des Öffnungsabschnitts 3 des Zylinders 2 bzw. in Fig. 8 nach oben mitgeschleppt. Bei einer Umkehr der Kolbenverschiebung in Ausstoßrichtung y, also in den Zylinder 2 hinein, werden die Sperrklinken 22 entriegelt und die Sperrhülse 19 verbleibt aufgrund ausreichend großen Reibschlusses mit der Innenwand 25 des in Fig. 8 oberen Zylinderabschnitts 46 in der Stellung lagefixiert, die sie bei der vorhergehenden Hubbewegung des Kolbens 12 bzw. der Kolbenstange 13 in Hubrichtung x erreicht hatte. Je nach Größe dieser Hubbewegung kann die Stellung, in der die Sperrhülse 19 durch Reibwirkung an der Innenwand 25 des in Fig. 8 oberen Zylinderabschnitts 46 festklemmt, unterschiedlich sein. In der äußersten, nämlich obersten Endstellung liegt die Sperrhülse 19 an der am Öffnungsabschnitt 3 des oberen Zylinderabschnitts 46 ausgebildeten Einziehung 33 an, die als Auszugssperre 35 dient und bewirkt, daß die Spritze nicht zerstörungsfrei demontiert werden kann. Bei der genannten Umkehr der Kolbenverschiebung in Ausstoßrichtung y gleiten die Sperrklinken 29 über die schiefwinklig angeordneten Zahnflanken 29 der Zähne 28 der Zahnung 27, ohne daß die Sperrhülse 19 verschoben wird. Bei einer erneuten Betätigung der Kolbenstange 13 in Hubrichtung x kann die Sperrhülse 19 durch die Klemmkraft der aufliegenden Sperrklinken 23 nur dann weiter in Richtung zur Einziehung 33 verschoben werden, wenn sie sich vorher im Abstand von dieser befand. Der Kolben 12 bzw. die Kolbenstange 13 kann somit nur einmal aus der in Fig. 8 dargestellten Ingebrauchnahmestellung über den vollen Kolbenhub in Hubrichtung x gezogen werden. Demnach ist auch sichergestellt, daß nur ein einziges Mal das maximal mögliche Volumen an gewünschter flüssiger Substanz ausgestoßen bzw. verabreicht werden kann.

Da z. B. bei der vorbeschriebenen Ausführungsform nach den Fig. 1 bis 6 unter gewaltsamer Einwirkung auf die Kolbenstange 13 in Hubrichtung es grundsätzlich denkbar ist, daß die Zähne 28 abschmirgeln und somit ihre Sperrwirkung in Verbindung mit den Sperrklinken 23 verlieren mit der Folge, daß eine Mehrfachverwendung der Spritze möglich ist, ist gemäß den Fig. 9 bis 11 zur Verhinderung dieser Möglichkeit vorgesehen, daß die Sperrelemente in Form der diametral angeordneten Arretierungszungen 22 samt Sperrklinken 23 jeweils über eine Sollbruchstelle 37 in Form einer Materialschwächung mit dem übrigen Teil der Sperrhülse 19 verbunden sind, wobei unmittelbar oberhalb der Sollbruchstelle 37 jeweils eine sich radial nach außen erstreckende Ausnehmung, hier Durchgang 34, angeordnet ist, in die bei Bruch der Sollbruchstelle 37 unter übermäßiger Einwirkung auf das Sperrglied 20 bzw. die Sperrhülse 19 in Sperrichtung dieses unter Herstellung einer irreversiblen, funktionszerstörenden Blockierstellung hineingedrängt wird, so wie dies in Fig. 9 mit unterbrochener Linie angedeutet ist (s. dazu auch Pfeil 48). In dieser Stellung erfolgt eine praktisch unlösbare Verklemmung zwischen der Zahnung 27 an der Kolbenstange 13 einerseits und der Sperrhülse 19 andererseits über das bzw. die "quergestellten" Arretierungszungen 22 samt Sperrklinken 23. Auf diese Weise wird eine irreversible Blockierung der Kolbenstange 13 erreicht. Um das Eindringen der losgebrochenen Arretierungszungen 22 in die radialen Ausnehmungen 34 zu erleichtern, ist der Querschnitt der Arretierungszungen 22 entsprechend Fig. 9 etwa S-förmig ausgebildet, wobei die Sperrklinken 23 jeweils zur Zahnung 27 der Kolbenstange 13 hin gerichtet sind. Fig. 9 zeigt übrigens einen Schnitt durch die Sperrhülse 19 längs Linie XIII-XIII in Fig. 10, d. h. einen Schnitt übereck.

Als zusätzliche Sicherheit gegen eine Mehrfachverwendung der Spritze ist bei der zuletzt genannten Ausführungsform zusätzlich oder alternativ vorgesehen, daß entsprechend Fig. 11 die Betätigungsscheibe 15 über eine ringförmige Sollbruchlinie 38 in Form einer Materialschwächungslinie mit dem oberen freien Kolbenstangenende 13 verbunden ist. Bei übermäßiger Einwirkung in Saughubrichtung, d. h. bei in Saughubrichtung x gesperrter Kolbenstange, bricht die Betätigungsscheibe 15 längs der Sollbruchlinie 38 von der Kolbenstange 13 ab, so daß eine Einwirkung auf die Kolbenstange 13 in Hubrichtung x nicht mehr möglich ist.

Die genannte Sollbruchlinie kann auch im oberen Bereich des Mittelstücks 8 der Kolbenstange 13 vorgesehen sein, und zwar als sich über den Umfang des Mittelstücks 8 der Kolbenstange 13 erstreckende Kerbung. Bei übermäßiger Einwirkung auf die Kolbenstange 13 würde dann der obere Teil der Kolbenstange 13 vom übrigen Teil desselben abbrechen, wobei die Sollbruchlinie dann vorzugsweise innerhalb des Zylinders 2 liegt, so daß nicht einmal mehr mittels Werkzeugen, wie Zangen oder dgl., auf die Kolbenstange eingewirkt werden kann.

Als Transportschutz sind noch gesonderte Abdeckkappen 42, 43 vorgesehen, von denen die eine über das rückwärtige Ende der Spritze unter Abdeckung der aus der Spritze herausragenden Kolbenstange samt Handhabe 14 und die andere über das vordere Ende der Spritze unter Abdeckung der in Fig. 8 nicht dargestellten Injektionsnadel stülpbar bzw. aufsteckbar ist.

In Fig. 14 ist eine weitere Ausführungsform einer erfindungsgemäß ausgebildeten Spritze 49 im schematischen Längsschnitt dargestellt, die ähnlich wie die Spritze gemäß Fig. 8 mit einem oberen Zylinderabschnitt 46 und einem unteren Zylinderabschnitt 45 kleineren Durchmessers ausgebildet ist. Von besonderem Interesse ist das Sperrglied 20, welches zwei oder mehr jeweils diametral zueinander angeordnete metallische Klemmzungen 41 entsprechend der Ausführungsform gemäß Fig. 7 aufweist. Die Klemmzungen 41 sind in die Sperrhülse 19 eingegossen und wirken mit ihren freien Enden wie anhand der Fig. 11 beschrieben auf die Kolbenstange 13 sperrend ein. Bei einer ungeraden Anzahl von Klemmzungen 41 sind diese vorzugsweise gleichmäßig über den Umfang der Kolbenstange verteilt angeordnet, die bei der Ausführungsform nach Fig. 14 einen kreisförmigen Querschnitt aufweist.

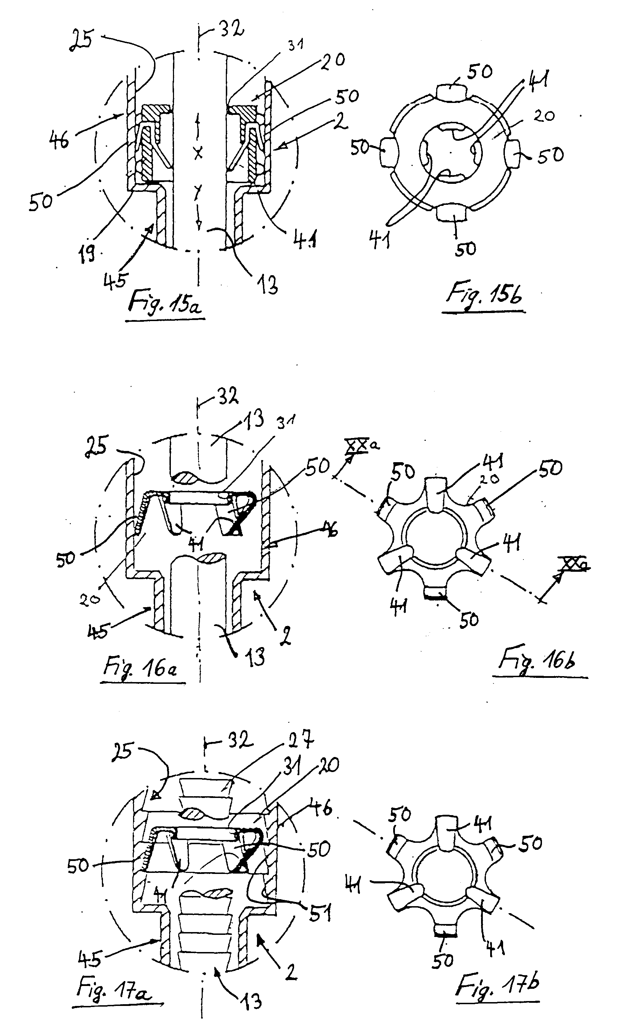

In Fig. 15a ist die Einzelheit "Z" in Fig. 14, nämlich das Sperrglied 20 in Zuordnung zum Spritzenzylinder 2 im Längsschnitt und vergrößertem Maßstab, nochmals hervorgehoben dargestellt. Demgemäß weist das Sperrglied 20 nicht nur mit der Kolbenstange 13 zusammenwirkende Klemmzungen 41 , sondern auch mit der Innenwand 25 des dem Sperrglied 20 zugeordneten Zylinderabschnitt 46 des Spritzenzylinders 2 zusammenwirkende Klemmzungen 50 auf. Die Klemmzungen 50 ersetzen die oben beschriebene Reibklemmung zwischen Sperrglied 20 und zugeordnetem Zylinderabschnitt bzw. Führungszylinder 46, d. h. bewirken die Lagefixierung des Sperrglieds 20 nach einem Saughub in Saughubrichtung "x". Die auf die Kolbenstange 13 einwirkenden Klemmzungen 41 sperren demnach die Kolbenstange 13 in Saughubrichtung "x", während die äußeren Klemmzungen 50 das Sperrglied 20 in Ausstoßrichtung "y" an der Innenwand 25 des zugeordneten Führungszylinders bzw. Zylinderabschnitts 46 festhalten. In Fig. 15b ist das Sperrglied 20 gemäß Fig. 15a in Draufsicht dargestellt.

Die Ausführungsform nach Fig. 16a, 16b,ist hinsichtlich der Wirkung mit derjenigen nach Fig. 15a, 15b,identisch. Es wird lediglich auf eine gesonderte Sperrhülse 19 verzichtet. Das Sperrglied 20 ist als einteiliges Stanz- und Biegeteil aus Metall hergestellt. Die der Kolbenstange 13 zugeordneten Klemmzungen 41 sowie der Innenwand 25 des Zylinderabschnitts 46 zugeordneten äußeren Klemmzungen 50 sind hinsichtlich ihrer Wirkung bzw. Funktion anhand der Fig. 15a, 15b bereits beschrieben. In Fig. 16b ist das Sperrglied 20 gemäß Fig. 16a in Ansicht von unten dargestellt, während die Darstellung des Sperrglieds 20 in Fig. 16a ein Querschnitt längs Linie XXa-XXa in Fig. 16b ist.

Von besonderem Interesse ist noch die Ausführungsform nach Fig. 17a, 17b, die durch ein einteiliges Sperrglied 20 entsprechend Fig. 16a, 16b gekennzeichnet ist, jedoch mit dem Unterschied, daß das Sperrglied 20 bei der Ausführungsform nach den Fig. 17a, 17b vorzugsweise aus Kunststoff hergestellt ist. Um trotzdem die beschriebene Funktionssicherheit in x- und y-Richtung zu gewährleisten, wirken die inneren und äußeren Klemmzungen 41 bzw. 50 mit entsprechenden bzw. komplementären Zahnungen an der Kolbenstange 13 einerseits und an der Innenwand 25 des dem Sperrglied 20 zugeordneten Zylinderabschnitts 46 des Spritzenzylinders 2 andererseits zusammen. Die Kolbenstangenzahnung 27 entspricht derjenigen nach den Fig. 1 bis 4. Die mit den äußeren Klemmzungen 50 des Sperrglieds 20 zusammenwirkende Zahnung an der Innenwand 25 des dem Sperrglied 20 zugeordneten Zylinderabschnitt 46 des Spritzenzylinders 2 ist in Fig. 17a mit der Bezugsziffer 51 gekennzeichnet. Sie umfaßt sich über den Umfang erstreckende Zähne mit sich jeweils rechtwinklig sowie schiefwinklig zur Kolben- bzw. Spritzenlängsachse 32 erstreckende Zahnflanken, wobei erstere der rückwärtigen Zylinderöffnung 3 und letztere dem unteren, dem Kolben 12 zugeordneten Zylinderabschnitt 45 zugewandt sind. In gleicher Weise ist die Zahnung 27 an der Kolbenstange 13 ausgebildet. Dementsprechend sperren die mit der Kolbenstangen-Zahnung 27 zusammenwirkenden inneren Klemmzungen 41 die Kolbenstange 13 in Saughubrichtung "x", während die äußeren Klemmzungen 15 das Sperrglied 20 in Ausstoßrichtung "y" an der Innenwand 25 des dem Sperrglied 20 zugeordneten Zylinderabschnitts 46 sperren. Die in den Fig. 15a bis 17b dargestellten Ausführungsformen von Sperrgliedern 20 sind also jeweils funktionsgleich. Die Ausführungsform nach den Fig. 17a, 17b hat gegenüber den vorbeschriebenen Ausführungsformen jedoch den Vorteil, daß aufgrund der formschlüssigen Zusammenwirkung zwischen Sperrglied 20 einerseits und Kolbenstange 13 bzw. Zylinder-Innenwand 25 andererseits keine Schlupfbewegungen auftreten.

Sämtliche in den Unterlagen offenbarten Merkmale werden als erfindungswesentlich beansprucht, soweit sei einzeln oder in Kombination gegenüber dem Stand der Technik neu sind.

## Patentansprüche

1. Spritze, insbesondere für den medizinischen Gebrauch, mit einem Zylinder (2), der an seinem vorderen Ende (5) eine Öffnung (7) aufweist und in dem ein Kolben (12) bewegbar ist, dessen Kolbenstange (13) aus dem rückwärtigen Ende des Zylinders (2) herausragt, wobei an der bzw. in die Öffnung (7) eine Nadel, insbesondere Injektionsnadel, oder dgl. Saug- und/oder Verabreichungsteil fluiddicht an- bzw. einsetzbar ist, und wobei der Kolbenstange (13) Sperrmittel zugeordnet sind, derart, daß das Spritzenvolumen nur ein einziges Mal verabreichbar ist,
dadurch **gekennzeichnet**,
daß das Sperrmittel (20) eine gesonderte, von der Kolbenstange (13) verschieblich durchsetzte und von dieser nur in Saughubrichtung (X) mitschleppbare Sperrhülse (19) ist, derart, daß diese einerseits bei Ausstoßbewegung (Y) der Kolbenstange (13) ohne Behinderung derselben an der beim Saughub (X) mitgeschleppten Stellung verharrt, insbesondere durch in Ausstoßrichtung (Y) wirksamen Reib- oder Formschluß zwischen Sperrhülse (19) und Innenwand (25) des Zylinders (2), andererseits jedoch eine erneute Saughubbewegung der Kolbenstange (13) durch Eingriff an der Kolbenstange (13) blockiert.

2. Spritze nach Anspruch 1,
dadurch **gekennzeichnet**,
daß die Sperrhülse (19) mindestens ein Sperrelement in Form einer mit je einer Sperrklinke (23) versehenen Arretierungszunge (22) oder in Form einer federelastischen Klemmzunge (41) aus Metall oder dgl. federelastischem Material aufweist.

3. Spritze nach Anspruch 1 oder 2,
dadurch **gekennzeichnet**,
daß die Kolbenstange (13) mit einer umlaufenden Zahnung (27) versehen ist, mit der sich die Arretierungszunge(n) (22) in mechanischem Eingriff befindet, derart, daß eine wiederholte Saughubbewegung der Kolbenstange (13) verhindert ist.

4. Spritze nach Anspruch 3,
dadurch **gekennzeichnet**,
daß die Zahnung (27) sägezahnartig mit Zähnen (28) gleichen Zahnprofils ausgebildet ist, wobei die jeweils eine Zahnflanke (29) der Zähne (28) schiefwinklig zur Kolbenlängsachse (32) und die jeweils andere Zahnflanke (30) der Zähne (28) rechtwinklig zur Kolbenlängsachse (32) angeordnet ist.

5. Spritze nach einem der Ansprüche 1 bis 4,
dadurch **gekennzeichnet**,
daß die Bewegung des Sperrglieds (20) in Saughubrichtung (x) durch einen Anschlag im Bereich des rückwärtigen Endes des Zylinders (2) begrenzt ist.

6. Spritze nach Anspruch 5,
dadurch **gekennzeichnet**,
daß im Bereich der rückwärtigen Öffnung (3) des Zylinders (2) eine Auszugssperre (35) für das Sperrglied (20) ausgebildet ist, die zugleich als Bewegungsanschlag für das Sperrglied (20) dient.

7. Spritze nach Anspruch 6,
dadurch **gekennzeichnet**,
daß die Auszugssperre (35) als den freien Querschnitt des Zylinders (2) verkleinernde Einziehung (33) der Innenwand (25) desselben ausgebildet ist.

8. Spritze, insbesondere nach Anspruch 6,
dadurch **gekennzeichnet**,
daß die Auszugssperre (35) als in der Innenwand (25) des Zylinders (2) angeordnete umlaufende Nut ausgebildet ist, in der ein am Sperrglied (20) ausgebildeter umlaufender Steg einrastbar ist.

9. Spritze nach einem oder mehreren der Ansprüche 1 bis 8,
dadurch **gekennzeichnet**,
daß im Bereich der rückwärtigen Öffnung (3) des Zylinders (2) ein Führungsteil (9) angeordnet ist, dessen Führungsbuchse (10) in den Zylinder (2) eingesetzt und durch die die Kolbenstange (13) hindurchgeführt ist.

10. Spritze nach einem der Ansprüche 1 bis 9,
**gekennzeichnet** durch eine am freien, aus dem Zylinder (2) herausragenden Ende der Kolbenstange (13) seitlich vorstehende Handhabe (14), insbesondere Betätigungsscheibe (15), wobei die Handhabe (15) über eine Sollbruchlinie (38), insbesondere in Form einer Materialschwächungslinie, mit der Kolbenstange (13) verbunden ist.

11. Spritze, insbesondere nach einem der Ansprüche 1 bis 10,
dadurch **gekennzeichnet**,
daß die Sperrmittel (19, 20, 22, 23) über Sollbruchstellen (37) verbundene Sperrelemente (22, 23) aufweisen, derart, daß sie beim Versuch, die sperrwirkung zu überwinden, abbrechen und in eine irreversible, funktionszerstörende Blockierstellung gedrängt werden.

12. Spritze nach Anspruch 11,
dadurch **gekennzeichnet**,
daß die als Sperrelement dienende(n) Arretierungszunge(n) (22) samt Sperrklinke (23) oder die als Sperrelement dienende Klemmzunge (41) über eine Sollbruchstelle (37), insbesondere in Form einer Materialschwächung, mit dem übrigen Teil des Sperrglieds (20) bzw. der Sperrhülse (19) verbunden ist.

13. Spritze nach Anspruch 12,
dadurch **gekennzeichnet**,
daß der Sollbruchstelle (37) eine sich radial nach außen erstreckende Ausnehmung (34) zugeordnet ist, in die bei Bruch der Sollbruchstelle (37) unter übermäßiger Einwirkung auf das Sperrglied (20) entgegen der Sperrwirkung desselben das bzw. die dem Sperrglied (20) zugeordneten Sperrelemente (22, 23; 41) unter Herstellung einer irreversiblen, funktionszerstörenden Blockierstellung hineindrängbar ist bzw. sind.

14. Spritze nach einem oder mehreren der Ansprüche 1 bis 13,
dadurch **gekennzeichnet**,
daß die Kolbenstange (13) im Bereich ihres rückwärtigen Endes bzw. im der Handhabe (14) zugewandten Bereich (16) eine Sollbruchstelle aufweist, insbesondere in Form einer sich über den Umfang der Kolbenstange (13) erstreckenden Kerbung, wobei die Sollbruchstelle bei in den Zylinder (2) eingeschobener Kolbenstange (13) vorzugsweise innerhalb desselben liegt.

15. Spritze nach einem oder mehreren der Ansprüche 1 bis 14,
dadurch **gekennzeichnet**,
daß das Sperrglied (20) ein einteiliges Bauteil mit mit der Kolbenstange (13) einerseits und der Innenwand (25) des Spritzenzylinders (2) andererseits zusammenwirkenden Klemmzungen (41 bzw. 50) ist derart, daß beim Saughub (x) unter Mitnahme des Sperrglieds (20) eine Sperrung zwischen Sperrglied (20) und Kolbenstange (13) und beim Ausstoßen (y) unter Lagefixierung des Sperrglieds (20) im Zylinder (2) eine Sperrung zwischen Sperrglied (20) und Zylinder bzw. Zylinder-Innenwand (25) erfolgt (Fig. 19 - 21).

## Claims

1. Syringe, in particular for medical use, having a barrel (2) which has an opening, (7) at its front end (5) and in which a plunger (12) can be moved, the plunger rod (13) of which projects from the rear end of the barrel (2), it being possible to fit on or into the opening (7) a needle, in particular an injection needle, or similar suction and/or administration component in a leakproof manner, and the plunger rod (13) being assigned blocking means in such a way that the syringe volume can be administered only once, characterized in that the blocking means (20) is a separate blocking sleeve (19) through which the plunger rod (13) passes with displacement and which can be carried by the latter only in the auction stroke direction (X), in such a way that this blocking sleeve (19) on the one hand in the event of an ejection movement (Y) of the plunger rod (13) remains, without obstructing the latter, at the position to which it is carried on the suction stroke (X), in particular by frictional or positive engagement effective in the ejection direction (Y) between the blocking sleeve (19) and the inner wall (25) of the barrel (2), but on the other hand blocks a renewed suction stroke movement of the plunger rod (13) by means of engagement on the plunger rod (13).

2. Syringe according to Claim 1, characterized in that the blocking sleeve (19) has at least one blocking element in the form of a locking tongue (22) provided with in each case one catch (23) or in the form of a resilient clamp tongue (41) made of metal or similar resilient material.

3. Syringe according to claim 1 or 2, characterized in that the plunger rod (13) is provided with a peripheral toothing (27) with which the locking tongue(s) (22) is/are in mechanical engagement, in such a way that a repeated suction stroke movement of the plunger rod (13) is prevented.

4. Syringe according to Claim 3, characterized in that the toothing (27) is designed in saw-tooth formation with teeth (28) of identical tooth profile, the one tooth edge (29) of the teeth (28) in each case being arranged at an oblique angle to the longitudinal axis (32) of the plunger, and the other tooth edge (30) of the teeth (28) being arranged in each case at right angles to the longitudinal axis (32) of the plunger.

5. Syringe according to one of Claims 1 to 4, characterized in that the movement of the blocking member (20) in the suction stroke direction (x) is limited by a stop in the area of the rear end of the barrel (2).

6. Syringe according to Claim 5, characterized in that there is arranged, in the area of the rear opening (3) of the barrel (2), an anti-removal block (35) for the blocking member (20), which serves at the same time as a movement stop for the blocking member (20).

7. Syringe according to Claim 6, characterized in that the anti-removal block (35) is designed as a constriction (33) of the inner wall (25) of the barrel (2), reducing the free cross-section of the said barrel.

8. Syringe, in particular according to Claim 6, characterized in that the anti-removal block (35) is designed as a circumferential groove, which is arranged in the inner wall (25) of the barrel (2) and in which a circumferential ridge formed on the blocking member (20) can engage.

9. Syringe according to one or more of Claims 1 to 8, characterized in that there is arranged in the area of the rear opening (3) of the barrel (2) a guide part (9), the guide bush (10) of which is inserted in the barrel (2) and through which the plunger rod (13) is guided.

10. Syringe according to one of Claims 1 to 9, characterized by a hand grip (14), in particular in activation disc (15), laterally protruding at the free end of the plunger rod (13) projecting from the barrel (2), the hand grip (15) being connected to the plunger rod (13) via a predetermined break line (38), in particular in the form of a line of weakened material.

11. Syringe, in particular according to one of Claims 1 to 10, characterized in that the blocking means (19, 20, 22, 23) have blocking elements (22, 23) connected via predetermined break points (37) in such a way that, in the event of an attempt being made to overcome the blocking action, they break off and are forced into an irreversible, function-inhibiting blocking position.

12. Syringe according to Claim 11, characterized in that the locking tongue(s) (22) serving as blocking elements together with the catch (23) or the clamp tongue (41) serving as blocking element are connected to the remaining part of the blocking member (20) or blocking sleeve (19) via a predetermined break point (37), in particular in the form of a material weakening.

13. Syringe according to Claim 12, characterized in that the predetermined break point (37) is assigned a radially outwardly extending recess (34) into which, upon rupture of the predetermined break point (37), and with excessive action being exerted on the blocking member (20) counter to the blocking action thereof, the blocking element or elements (22, 23; 41) assigned to the blocking member (20) can be forced, producing an irreversible, function-inhibiting blocking position.

14. Syringe according to one or more of Claims 1 to 13, characterized in that the plunger rod (13) has, in the area of its rear end or in the area (16) facing the hand grip (14 ), a predetermined break point, particularly in the form of a notch extending round the circumference of the plunger rod (13), in which case the predetermined break point, when the plunger rod (13) is inserted into the barrel (2), preferably lies inside the latter.

15. Syringe according to one or more of Claims 1 to 14, characterized in that the blocking member (20) is a one-part component with clamp tongues (41 and 50) cooperating on the one hand with the plunger rod (13) and on the other hand with the inner wall (25) of the syringe barrel (2), in such a way that, on the suction stroke (x), with the blocking member (20) being carried along, there is a blocking between blocking member (20) and plunger rod (13) and, on ejection (y), with positional fixing of the blocking member (20) in the barrel (2), there is a blocking between blocking member (20) and barrel or inner wall (25) of barrel (Figures 19-21).

## Revendications

1. Seringue, à usage médical notamment, avec un cylindre (2), qui présente une ouverture (7) sur son extrémité avant (5) et dans lequel peut se déplacer un piston (12), dont la tige (13) dépasse de l'extrémité arrière du cylindre (2), une aiguille, une aiguille à injections notamment, ou autre élément d'aspiration et/ou d'administration, pouvant être fixée et/ou logée, d'une manière étanche aux fluides, sur et/ou dans l'ouverture (7), et des moyens d'arrêt étant associés à la tige de piston (13), de sorte que le volume de la seringue ne peut être administré qu'une seule fois, caractérisée en ce que le moyen d'arrêt (20) est une douille d'arrêt (19) séparée, traversée avec une possibilité de déplacement par la tige de piston (13), par laquelle elle peut être entraînée dans le seul sens de la course d'aspiration (X), de sorte que cette douille demeure, lors du mouvement d'éjection (Y) de la tige de piston (13) et sans entraver cette dernière, dans la position entraînée lors de la course d'aspiration (X), par suite notamment du blocage mécanique et de la friction, s'exerçant dans le sens d'éjection (Y), entre la douille d'arrêt (19) et la paroi interne (25) du cylindre (2), d'une part, mais qu'elle bloque un nouveau mouvement de course d'aspiration de la tige de piston (13), par sa mise en prise sur cette tige (13), d'autre part.

2. Seringue suivant la revendication 1, caractérisée en ce que la douille d'arrêt (19) présente un élément d'arrêt au moins, en forme d'une languette de blocage (22) munie d'un cliquet d'arrêt (23), ou en forme d'une languette de serrage élastique (41) en métal ou autre matériau élastique.

3. Seringue suivant l'une des revendications 1 et 2, caractérisée en ce que la tige de piston (13) est munie d'une denture périphérique (27), avec laquelle la (les) languette(s) de blocage (22) se trouve(nt) en prise mécanique, de sorte qu'un mouvement de course d'aspiration réitéré de la tige de piston (13) est entravé.

4. Seringue suivant la revendication 3, caractérisée en ce que la denture (27) est réalisée en dents de scie avec des dents (28) de même profil, chacun des flancs de dent (29) des dents (28) étant disposé à angle oblique par rapport à l'axe longitudinal (32) du piston, et chacun des autres flancs de dent (30) des dents (28) étant disposé à angle droit par rapport à l'axe longitudinal (32) du piston.

5. Seringue suivant l'une quelconque des revendications 1 à 4, caractérisée en ce que le déplacement de l'organe d'arrêt (20) est limité, dans le sens de la course d'aspiration (X), par une butée prévue dans la zone de l'extrémité arrière du cylindre (2).

6. Seringue suivant la revenditation 5, caractérisée en ce qu'un verrouillage de sortie (35) pour l'organe d'arrêt (20), qui sert simultanément de butée de déplacement pour l'organe (20), est réalisé dans la zone de l'ouverture arrière (3) du cylindre (2).

7. Seringue suivant la revendication 6, caractérisée en ce que le verrouillage de sortie (35) est réalisé sous forme de rétreint (33), réduisant la section libre du cylindre (2), de la paroi interne (25) de ce dernier.

8. Seringue, suivant la revendication 6 notamment, caractérisée en ce que le verrouillage d'arrêt (35) est réalisé sous forme de rainure périphérique, disposée dans la paroi interne (25) du cylindre (2), et dans laquelle peut s'enclencher un profil périphérique, réalisé sur l'organe d'arrêt (20).

9. Seringue suivant l'une quelconque ou plusieurs des revendications 1 à 8, caractérisée en te qu'une pièce de guidage (9), dont la douille de guidage (10) est logée dans le cylindre (2) et est traversée par la tige piston (13), est disposée dans la zone de l'ouverture arrière (3) du cylindre (2).

10. Seringue suivant l'une quelconque des revendications 1 à 9, caractérisée par une prise (14), un disque d'actionnement (15) notamment, en saillie latérale sur l'extrémité libre, dépassant du cylindre (2), de la tige de piston (13), la prise (15) étant assemblée avec la tige de piston (13) par l'intermédiaire d'une ligne de rupture (38), en forme d'une ligne d'affaiblissement de matériau, notamment.

11. Seringue, suivant l'une quelconque des revendications 1 à 10 notamment, caractérisée en te que les moyens d'arrêt (19, 20, 22, 23) présentent des éléments d'arrêt (22, 23), assemblés par l'intermédiaire de points de rupture (37), de sorte qu'ils se rompent, en tas de tentative de surmonter l'effet de blocage, et sont poussés dans une position de blocage irréversible, anéantissant le fonctionnement.

12. Seringue suivant la revenditation 11, caractérisée en ce que la (les) languette (s) de blocage, servant d'élément d'arrêt, avec le cliquet d'arrêt (23), ou la languette de serrage (41), servant d'élément d'arrêt, sont assemblés avec la partie restante de l'organe d'arrêt (20) et/ou de la douille d'arrêt (19) par l'intermédiaire d'un point de rupture (37), en forme d'un affaiblissement de matériau, notamment.

13. Seringue suivant la revenditation 12, caractérisée en ce qu'un évidement (34), s'étendant dans le sens radial externe, est associé au point de rupture (37), le et/ou les éléments d'arrêt (22, 23; 41), associé(s) à l'organe d'arrêt (20), pouvant être poussé(s) dans cet évidement, en créant une position de blocage irréversible, anéantissant le fonctionnement, en cas de rupture du point de rupture (37) par une action excessive sur l'organe d'arrêt (20), contre l'effet de blocage de ce dernier.

14. Seringue suivant l'une quelconque ou plusieurs des revendications 1 à 13, caractérisée en ce que la tige de piston (13) présente, dans la zone de son extrémité arrière et/ou dans la zone (16) tournée vers la prise (14), un point de rupture, en forme d'une entaille s'étendant sur le pourtour de la tige de piston (13), notamment, le point de rupture, en position de rentrée de la tige de piston (13) dans le cylindre (2), se situant de préférence à l'intérieur de ce dernier.

15. Seringue suivant l'une quelconque ou plusieurs des revendications 1 à 14, caractérisée en ce que l'organe d'arrêt (20) est un composant monobloc, avec des languettes de serrage (41 et/ou 50) concourant avec la tige de piston (13), d'une part, la paroi interne (25) du cylindre (2) de la seringue, d'autre part, de sorte que, lors de la course d'aspiration (X), un blocage se produit entre l'organe d'arrêt (20) et la tige de piston (13), avec un entraînement de l'organe d'arrêt (20), un blocage se produisant entre l'organe d'arrêt (20) et le cylindre et/ou la paroi interne (25) du cylindre lors de l'éjection (Y), avec une fixation de position de l'organe d'arrêt (20) dans le cylindre (2) (figures 19 - 21).
